# EUROPÄISCHE PATENTANMELDUNG

(11) **EP 4 799 593 A1**
(43) Veröffentlichungstag der Anmeldung: **02.09.2026**
(21) Anmeldenummer: 26155787.0
(22) Anmeldetag: 02.02.2026
(51) Int. Cl.: A61F 5/01, A61F 5/05, A61F 5/10

(54) **FINGERORTHESE UND VERFAHREN ZU IHRER HERSTELLUNG**

(30) Priorität: 27.02.2025 DE 102025107392
(71) Anmelder: NKM Tec GmbH, 07330 Probstzella (DE)
(72) Erfinder: Glaser, Volker, 07330 Probstzella (DE)
(74) Vertreter: Donath, Dirk

(57) **Zusammenfassung**

Die Erfindung betrifft eine Fingerorthese und Verfahren zu ihrer Herstellung.

Die Aufgabe der vorliegenden Erfindung, eine Fingerorthese, anzugeben, welche die Nachteile des Standes der Technik vermeidet, insbesondere patientenspezifisch ist, wird dadurch gelöst, dass diese eine, mindestens an einem Finger anbringbare, gegenüber den Fingern längs verlaufende Schiene (1), sowie zwei Bänder (2) mit einen Verschlussmechanismus (3) umfasst, wobei
- die Breite der Schiene (1) kürzer als ein Fingerglied ist und einen Steg (1') ausbildet, der zwischen zwei Fingern anordenbar ist sowie länger als zwei Fingerbreiten ist,
- der Steg (1') aus Kunststoff besteht und im Bereich seiner beiden freien Enden dichotom in jeweils zwei Enden übergeht, die jeweils zwei, sich gegenüberliegende Ausnehmung oder Laschen (11) aufweisen,
- die Bänder (2) aus elastischem, zugbeständigem Kunststoff oder Gummi bestehen und den Verschlussmechanismus (3) tragen, in dem die Bänder (2) auf einer Seite der Oberfläche mit den Noppen oder den Einkerbungen (21) versehen sind und die Form der freien Enden der Bänder (2) korrespondierend zu dem Ausnehmungen oder Laschen (11) sind, mittels derer sie aufnehmbar und mithilfe der Noppen oder Einkerbungen (21) fest arretierbar und wieder lösbar sind, wobei je ein Band (2) jeweils durch die gegenüberliegenden Ausnehmung oder Laschen (11) eines der zwei freien Enden des Stegs (1') verläuft sowie fest arretier- und wieder lösbar ist, in dem es an einem seiner freien Enden gegenüber einer der Ausnehmungen oder Laschen (11) geblockt ist,
so dass im aufgenommenen Zustand jedes Bandes (2) verstellbare Ösen (4) zur Aufnahme eines oder mehrerer Finger vorausgebildet sind, welche durch ein Bewegen der Bänder (2) in den Ausnehmungen oder Laschen (11) in ihrem Durchmesser veränderbar sind.

## Beschreibung

Die Erfindung betrifft eine Fingerorthese und Verfahren zu ihrer Herstellung, insbesondere eine Frühmobilisations- Blockungs- und Entlastungsschiene der Finger, bspw. als Relative Motion Splint (RMS).

Orthesen an sich sind medizinische Hilfsmittel, die Patienten kurzfristig oder dauerhaft zum Einsatz kommen. Hauptgründe für ihren Einsatz können angeborene Fehlstellungen, Lähmungen, Unfälle, Operationen oder altersbedingte Verschleißerscheinungen am Haltungs- und Bewegungsapparat sein und werden als maßgefertigte Produkte, Halbfertigprodukte oder Fertigprodukte hergestellt und angeboten.

Finger- und Handorthesen umschließen bspw. einzelne Finger oder die Hand, dynamische oder statische Fingerschienen, Fingerkappen, Daumenschienen oder Handtellerschutz, wobei diese aus verschiedenen Materialien, wie bspw. Kunststoff oder Faserverbundwerkstoff bestehen können.

Fingerorthesen werden oft verwendet, um Verletzungen wie Sehnenrisse, Arthritis, Frakturen oder Gelenkprobleme zu behandeln. Fingerorthesen können individuell angepasst oder vorgefertigt sein und dienen dazu, die Funktionalität der Hand und Finger zu verbessern/erhalten und Schmerzen zu lindern.

Bei den Fingerorthesen kennt man die RM-Orthese als eine fingerbasierte Mobilisationsorthese, die den betroffenen Finger oder das entsprechende Metacarpophalangealgelenk / den Metacarpophalangealjoint (MCPJ) des Fingers in einer etwas stärkeren Streckung oder Beugung positioniert als die benachbarten MCPJs, die als Relative Motion Extension (RME) bzw. Relative Motion Flexion (RMF) Orthesen bezeichnet werden (Schwartz DA. Relative motion orthoses: fabrication tips. J Hand Ther. 2023 Apr-Jun; 36(2):486-493. doi: 10.1016/j.jht.2022.12.004. Epub 2023 Apr 7. PMID: 37032245.)

Die Relative Motion Orthosis (RMO) oder Relative Motion Splint (RMS) wurde erstmals als Bestandteil eines postoperativen Rehabilitationsprotokolls für Strecksehnenverletzungen im Rahmen des ICAM-Schienenprogramms ("Immediate Active Controlled Motion Program) populär. Dieses wurde in den 1980er Jahren entwickelt und ist dafür bekannt, günstige Ergebnisse bei der Behandlung von Strecksehnenreparaturen von Zone IV bis Zone VII zu erzielen. (Mottay, N. & Govender, P. & Mpanza, D. (2020)

Das ICAM-Schienenkonzept bedeutet eine sofortige kontrollierte aktive Mobilisierung (ICAM) zu erzielen. Diese Technik schützt die reparierte Sehne, indem sie die Spannung auf sie verringert und den verletzten Finger mithilfe einer Jochschiene in relativer Hyperextension zu den unverletzten Fingern positioniert (https://www.orfit.com/physicalrehabilitation/blog/splint-in-the-spotlight-relative-motion-orthosis).

Es gibt unterschiedliche Ausführungen des RMS nach Jensen (Jensen, Carina 2021. Das Handschienen - Manual. Therapeutische Schieneversorgung - Neu & optimiert. 2. erweiterte Auflage; Wertvoll Wachsen Verlag Gießen):

### Relativ-Motion-Extension Splint (RMES):

Der bzw. die betroffene/n Finger (MCP-Gelenke) wird bzw. werden in 20 Grad Extension (Streckung) gegenüber den/m nicht-betroffenen Finger/n gelagert.

Anwendungsbereich (Beispiele):
- kontrollierte aktive Mobilisierung nach Sehnenreparatur durch Spannungsverringerung
- Entlastung entzündeter und gereizter Strecksehnen

### Relativ-Motion-Flexion Splint (RMFs):

Der bzw. die betroffene/n Finger (MCP-Gelenke) wird bzw. werden in 20 Grad Flexion (Beugung) gegenüber den/m nicht-betroffenen Finger/n gelagert.

Ein Beispiel für den Anwendungsbereich von RMFs ist der Einsatz als MCP-Blockungsspange bei Streckdefiziten im PIP-Gelenk mit folgenden Parametern:
- Beugung der MCP-Gelenke und Blockung im Rahmen der "normalen", alltäglichen Funktionalität der Hand
- verhindert somit die Überdehnung bzw. Überstreckung von den MCP's
- Folge: größerer Bewegungsradius in den PIP-Gelenken (Kontrakturvermeidung)
- Einsatz bei sämtlichen Aktivitäten des täglichen Lebens machbar (bei Selbstversorgung, Haushalt, Hobbys und Sport)

Derzeit sind verschiedene Fertigungsmethoden zu Herstellung der zuvor stehend genannten Fingerorthesen bekannt.

Die Materialien zur hand- und teilweise maschinell- gefertigten Herstellung sind insbesondere thermoplastische Strick- oder Wirkgewebe mit ohne oder mit Formgedächtnisfunktion. Beispielhaft dafür sind folgende Materialen mit ihren Eigenschaften genannt:
I. Orfit "Orficast" Schienenmaterial
   - thermoplastisches Strickgewebe auf einer z.B.: 3-Meter-Rolle
   - in verschiedenen Breiten, Stärken und Farben erhältlich
   - Elastizität in zwei Richtungen/Formgedächtnis (in Längsrichtung)
   - leicht zu schneiden
   - selbsthaftend, insbesondere bei Verwendung trockener Hitze
   - atmungsaktives Gewebe
   Quelle: https://krewi.de/produkte/verbaende/orficast/)
II. Diverses Thermoplastisches Schienenmaterial, wie bspw. Turbocast, Aquaplast, Manosplint, Orfit, AFH Schienenmaterial, Ezeform, oder Encore
   - thermoplastische Schiene = ist orthopädisches Hilfsmittel, dass zur Stabilisierung, Unterstützung und Immobilisierung von verletzten oder geschwächten Körperteilen verwendet wird
   - besteht aus einem speziellen Kunststoffmaterial, das bei Erwärmung (zwischen 60 und 75 Grad Celsius) flexibel und formbar wird, sodass es sich an die anatomischen Gegebenheiten des Patienten anpassen lässt
   - nach dem Abkühlen verhärtet die Schiene und behält ihre angepasste Form bei, Ermöglichung einer effektiven und individuell zugeschnittenen Stabilisierung
   mit folgenden wählbare Materialeigenschaften:
   1. Perforationstypen: nicht perforiert, mikroperforiert oder miniperforiert - Perforationen beeinflussen die Atmungsaktivität und Flexibilität der Schiene
   2. Beschichtungen: bspw. beidseitig flauschige Beschichtungen für Tragekomfort und Reduktion von Hautirritationen
   3. Memory-Effekt: ermöglicht, die Schiene mehrfach zu formen und anzupassen (bei progressiver Rehabilitation nützlich)
   4. Farben und Größen: beige, gelb, pink, hellblau und weiß und Größen (z.B. 45 cm x 60 cm oder 60 cm x 45 cm) (unterschiedliche Anforderungen/Vorlieben)
      (Quelle: https://premium-therapie.de/de/schienenmaterial/thermoplastisches-schienenmaterial? next_page=2)
III. Weitere Schienenmaterialien sind bspw.:
   - Faserverstärkte Kunststoffe: z.B. GFK (glasfaserverstärkte Kunststoffe) und CFK (kohlefaserverstärkte Kunststoffe) = Kombination aus Leichtigkeit und Festigkeit → ideal für den Einsatz in der sportmedizinischen Anwendung
   - Gipse und synthetische Alternativen (z.B. Kunstharze): stabile und formbare Unterstützung, Stabilität entsteht nach dem Aushärten des Materials → Metalle: Aluminium, Edelstahl und Titan für größere Festigkeit z.B. bei schweren Verletzungen
   (Quelle: https://premium-therapie.de/de/schienenmaterial/thermoplastisches-schienenmaterial? next_page=2)

Die dazugehörige Fertigungsmethode für angepasste Hook Splint Schlaufen zur Entlastung der Strecksehne am Mittelfinger sind im Internet unter: https://hooksplint.com/ offenbart und weisen folgende Merkmale auf:
→ glattes Silikonband mit Metallkern
→ flexibel anpassbar
→ wasserdicht

Der HOOK SPLINT als Relative Bewegungsorthese wird bspw. folgenden Schriften offenbart:
US 000004600618 A offenbart ein Schienenmaterial umfassend eine Folie aus thermoplastischem Material, die sich leicht verformen lässt, wenn sie auf eine erhöhte Temperatur erwärmt wird, und eine Schicht aus Schlingenflor-Material, die mit einer Oberfläche der Folie verbunden ist, so dass ein Riemen mit einer Vielzahl von Haken daran mechanisch an das Schlingenmaterial geklebt und von diesem gelöst werden kann.

Diese technische Lösung hat den Nachteil, dass durch die Haken eine Verletzungsgefahr besteht.

FR 000002860707 A1 offenbart eine Schiene mit einer durchgehenden Unterarm-Rückenwand und einer ventralen Fingerauflage. Die Wand ist mit zwei Klettverschlussbändern aus Samt versehen, denen gegenüber ein Klettverschlussstreifen mit Klebehaken angebracht ist. Ein weiterer Streifen ist in Höhe des Handgelenks angebracht. Der Daumen ist in Höhe des Handgelenks mit einer Niete an der Wand befestigt. Eine Baugruppe aus einer Einsteckklammer und einer Hakenbefestigung bildet dazu den Fixierungspunkt des Daumens.

Diese technische Lösung hat den Nachteil, dass sich der Klettverschlussstreifen mit Klebehaken lösen können und durch die Klebehaken eine gewisse Verletzungsgefahr besteht.

Das Gebrauchsmuster CN 000214805854 U auf dem technischen Gebiet der fadenklebenden Rückhaltegurte offenbart einen fadenklebenden Rückhaltegurt für eine Kinderzahnschiene, der einen ganzen fadenklebenden Rückhaltegurtkörper, einen Spannungsring und einen Abstandshalterblock umfasst, wobei eine Befestigungsplatte fest mit dem Boden des ganzen fadenklebenden Rückhaltegurtkörpers verbunden ist, eine Hülsenschale fest mit einer Seite der Befestigungsplatte verbunden ist und der Abstandshalterblock fest mit der Innenseite der Hülsenschale verbunden ist. Elastische Elemente sind mit den Innenseiten der Trennblöcke in einem eingebetteten Modus verbunden, Wickeltrommeln sind fest mit den Vorderseiten der elastischen Elemente verbunden, Befestigungsstreifen sind fest mit den beiden Enden der Befestigungsplatte verbunden, und die Befestigungsstreifen können es einem Benutzer ermöglichen, den Abstand zwischen den beiden Sätzen von Druckstäben in den Befestigungsstreifen entsprechend dem Körperbau eines Kindes anzupassen. Die Druckstange wird dabei durch den gegenseitigen Eingriffseffekt einer Positionierungszahnnut und eines Positionierungsblocks befestigt, wobei der Rückhaltemechanismus einem Benutzer ermöglicht, einen Baumwollgurt in der entsprechenden Länge herauszuziehen und den Baumwollgurt an einer ersten Klebeplatte zu befestigen.

Diese technische Lösung hat den Nachteil, dass sie nur für Zahnschienen vorgesehen ist und daher nicht auf Handorthesen übertragbar ist.

Das Gebrauchsmuster CN 000213851309 U offenbart eine mehrteilige Befestigungsschiene vom Spleisstyp mit einem magischen Klebeband-Fixierband, das eine Kunststoffschiene vom Spleisstyp und ein magisches Klebeband-Fixierband umfasst, wobei die Kunststoffschiene vom Spleisstyp beweglich mit dem magischen Klebeband-Fixierband verbunden ist und die Kunststoffschiene vom Spleisstyp eine äußere Seitenfläche, eine innere Seitenfläche, einen konvexen Schnallennagel, ein rundes Loch und eine Verstärkungsrippe umfasst, die Kunststoffklemmplatte des Spleisstyps ist bogenförmig, die nach außen vorstehende Fläche der Bogenform ist die Außenseitenfläche, die nach innen konkave Fläche der Bogenform ist die Innenseitenfläche, zwei Reihen von vorstehenden Schnallennägeln sind an einem Ende der Außenseitenfläche verteilt, zwei Reihen von runden Löchern sind in dem anderen Ende der Außenseitenfläche ausgebildet, und das Klettverschlussbefestigungsband besteht aus einem Klebeband und einer Schnalle. Die thermoplastische Kunststoffschiene des Spleisstyps und das magische Klebeband-Fixierband werden kombiniert, um die Fixierschiene zu bilden, Formung und Knoten sind nicht erforderlich, Materialien werden flexibel für die Fixierung entsprechend der Position und Länge einer Wunde ausgewählt, die Fixierfestigkeit, Festigkeit und Stützkraft werden verbessert, die Fixierschiene deckt die Wunde ab, sekundäre Verschmutzung der Wunde wird verhindert, und der Fixierzeitverbrauch wird verkürzt.

Diese technische Lösung hat den Nachteil, dass durch den Schnallennagel eine Verletzungsgefahr besteht.

Der HOOK SPLINT als Relative Bewegungsorthese ist somit ein Medizinprodukt der neueren Generation und dient der professionellen Versorgung von Strecksehnenverletzungen am Handrücken sowie am Unterarm zur Handrehabilitation, von Verletzungen, Beugungen, Kontrakturen, Dehnungen, Frakturen etc. und findet speziell für Patienten mit abnormaler Beweglichkeit des PIP-Gelenkes (verschiedene Diagnosen) Anwendung
(Quelle: https://mebocare.com/de/produkt/hook-splint/? srsltid=AfmBOopHsRcOHpovprO2IPRqdJOr0it8mEiOlrhdDJ9oV_C_84 g-jb4r)

Beim bekannten WE - Design Silversplints: RMS - Relative Motion Splint 5049 ist es das Ziel, einen herabhängenden, "herabfallenden" Finger verhindern. Bei der Streckung wird dabei der hängende Finger von den beiden benachbarten Fingern getragen/angehoben. Anwendung findet diese technische Lösung insbesondere bspw. bei Herabhängen des Fingers, Strecksehnenverletzung und Fingerscheren. Durch die höhere Platzierung der Mittelstütze dieser Schiene entsteht dabei ein sogenannter "Relative Motion Splint" (Yoke), der bei Strecksehnenverletzungen eingesetzt werden kann.
(Quelle: https://silversplints.de/modelle/rms-relative-motion-splint-5049)

Im Folgenden werden die Möglichkeiten, Probleme sowie Grenzen der derzeitigen Materialien und Modelle aufgeführt.
1. Thermoplastische Materialien
   - nur für den kurzzeitigen Einsatz geeignet
   - müssen individuell angepasst werden, Vorgang benötigt mehr Zeit als bei Fertigmodellen z.B. zur Installation und zum Aushärten
   - benötigen Zugang zu heißem Wasser und/oder Heißluft → Strom/Energie zum Modellieren
   - sind kurzlebig (werden spröde und brüchig)
   - sind temperaturempfindlich (werden durch äußere Einflüsse wie höhere Temperaturen in der Formgebung beeinflusst)
   - oft ohne Verschlusssysteme - schwer modulier- und anpassbar, besonders bei Veränderungen der Hand/Finger (Umfang)
   - sind oft sperrig, unbequem und zu starr
   - sind oft schwer auszuziehen, schwer zu reinigen und trocken zu halten
2. Hook Splint
   - unbequem beim längeren Tragen → Material und Perforation erzeugt Druckstellen, zu hart auf der Fingerhaut (Bereich Blockung)
   - Gummierung reibt sich nach der Zeit ab → unterliegendes Metall erzeugt eine Verletzungsgefahr für die Haut
   - Winkelgrade der erwünschten Beugung und Streckung im MCP - Gelenk nur bedingt einstellbar
   - nur schwer anwendbar für äußere Finger wie Kleiner Finger und Zeigefinger
   - nur bedingt anwendbar bei mehreren betroffenen Fingern
   - Verschlusssystem schwer umsetzbar für Patienten mit eingeschränkter Feinmotorik
   - ästhetisch nicht besonders ansprechend, keine Farbauswahl möglich
   - nur bedingt bei sportlichen oder schweren Arbeiten einsetzbar (insbesondere wegen der Verletzungsgefahr durch den Metallkern)
3: We - Design Silversplints: (RMS - Relative Motion Splint 5049)
   - unbequem beim längeren Tragen → Material erzeugt Druckstellen, zu hart auf der Fingerhaut (Blockung)
   - schwer justierbar/biegbar aufgrund der Materialhärte - Finger werden vermessen, Material wird nicht direkt auf der Haut angepasst → Passform fehleranfällig
   - Winkelgrade der erwünschten Beugung und Streckung im MCP - Gelenk nur bedingt einstellbar
   - kein Verschlusssystem - rutscht und ist kaum justierbar bei veränderten Fingerumfängen
   - hoher Preis
   - nur über einen Kostenvoranschlag bei der Krankenkasse einzureichen (Sanitätshauspartner) → lange Wartezeiten bis zur Genehmigung/Herstellung für den Patienten
   - nicht einzusetzen bei schweren Arbeiten oder sportlichen Aktivitäten - Verletzungsgefahr

Die Aufgabe der vorliegenden Erfindung besteht darin, eine Fingerorthese und ein Verfahren zu ihrer Herstellung anzugeben, welche die Nachteile des Standes der Technik vermeiden, insbesondere eine flexibel, multifunktional Fingerorthese mit modulare Eigenschaften bereit zu stellen, welche preiswert ist und gleichzeitig hohen und individuell anpassbaren Tragekomfort gewährleistet sowie robust, langlebig, leicht und optisch ansprechend ist.

Des Weiteren soll die bereitzustellende Fingerorthese die Versorgung von einzelnen oder mehreren Fingern ermöglichen, für Patient und Therapeut einfach zu installieren sein, nach dem Anlagen für den Patienten bequem sein, wobei sie nicht rutschen darf und gleichzeitig / trotzdem flexibel, stützend und leicht dehnbar sein muss.

Erfindungsgemäß wird diese Aufgabe durch die Merkmale des ersten Patentanspruchs gelöst. Weitere günstige Ausgestaltungsmöglichkeiten der Erfindung sind in den nachgeordneten Patentansprüchen angegeben.

Die bereitgestellte Fingerorthese geht von folgendem vorbekannten Stand der Technik aus:
Eine Fingerorthese gemäß dem Stand der Technik umfasst eine an der Ober- oder Unterseite eines Fingers anbringbare, zu dem Finger längs verlaufende Schiene, deren Länge der Länge des Fingers entspricht sowie zwei Bänder mit einem Verschlussmechanismus, wobei die Schiene aus Kunststoff ist und im Bereich ihrer freien Enden über jeweils zwei, sich gegenüber liegende Ausnehmung verfügt, und die Bänderus elastischem, zugbeständigem Kunststoff oder Gummi bestehen und einen Verschlussmechanismus ausbilden, in dem die Ausnehmungen in ihren Form korrespondierend zu den Bändern sind und mittels derer die Bänder aufgenommen werden, in dem je ein Band jeweils durch die zwei gegenüber liegenden Ausnehmung eines freien Endes der Schiene verläuft sowie fest arretier- und wieder lösbar ist, in dem das Band an einem seiner freien Enden gegenüber einer der Ausnehmungen geblockt ist.

Ausgehend von diesem Stand der Technik werden drei Varianten einer neuen Fingerorthese bereitgestellt.

Die bereitgestellte Fingerorthese umfasst eine, mindestens an einem Finger anbringbare, gegenüber den Fingern längs verlaufende Schiene, sowie zwei Bänder mit einen Verschlussmechanismus, welche gegenüber dem Stand der Technik wie folgt abgewandelt sind:
Die Breite der Schiene [welche der Länge der Schiene gemäß dem Stand der Technik entspricht] ist kürzer als ein Fingerglied und bildet einen Steg aus, der zwischen zwei Fingern anordenbar ist sowie länger als zwei Fingerbreiten ist.

Dieser Steg besteht aus Kunststoff und geht im Bereich seiner beiden freien Enden dichotom in jeweils zwei Enden über, die jeweils zwei, sich gegenüberliegende Ausnehmung oder Laschen aufweisen, wobei die Bänder aus elastischem, zugbeständigem Kunststoff oder Gummi bestehen und den Verschlussmechanismus tragen, in dem die Bänder auf einer Seite der Oberfläche mit den Noppen oder den Einkerbungen versehen sind und die Form der freien Enden der Bänder korrespondierend zu dem Ausnehmungen oder Laschen sind, mittels derer sie aufnehmbar und mithilfe der Noppen oder Einkerbungen fest arretierbar und wieder lösbar sind (s.g. Klicksystem).

Dabei verläuft je ein Band (2) jeweils durch die gegenüberliegenden Ausnehmung oder Laschen (11) eines der zwei freien Enden des Stegs (1') und ist fest arretier- und wieder lösbar, in dem es an einem seiner freien Enden gegenüber einer der Ausnehmungen oder Laschen (11) geblockt ist,

Dadurch werden im aufgenommenen Zustand jedes Bandes (2) verstellbare Ösen (4) zur Aufnahme eines oder mehrerer Finger vorausgebildet, welche durch ein Bewegen der Bänder (2) in den Ausnehmungen oder Laschen (11) in ihrem Durchmesser veränderbar sind.

Dabei kann der Steg etwas schmaler ausgeführt und mit einer Mulde zur Auflage von einem Finger ausgeführt sein, oder etwas breiter ausgeführt und mit zwei Mulden zur Auflage von zwei Fingern ausgeführt sein.

Alternativ dazu kann der Steg und ein Band oder der Steg und zwei Bänder als Doppelschlaufe für zwei Finger oder Einfachschlaufe für einen Finger wie folgt ausgeführt sein:
Diese beiden Alternativen der Fingerorthese umfassen eine, an einem oder zwei Fingern anbringbare, gegenüber den Fingern längs verlaufende Schiene, sowie ein oder zwei Bänder mit einen Verschlussmechanismus, wobei die Breite der Schiene kürzer als ein Fingerglied ist und einen Steg ausbildet, der länger als zwei Fingerbreiten ist und der Steg zwischen zwei Fingern längs verlaufend zu den Fingern anordenbar ist und ein- oder beidseitig in ein Band mit einem Verschlussmechanismus übergeht.

Dabei sind der Steg und das Band in der ersten Ausgestaltung einstückig und bestehen aus Kunststoff, wobei der Steg mittig angeordnet ist und über eine Ausnehmung oder zwei Ausnehmungen verfügt sowie gegenüber dem Band in seiner Konsistenz fester und härter ist.

Dabei ist das Band elastisch sowie zugbeständig und trägt über zumindest einen Teil der Gesamtlänge zumindest auf einer Seite auf seiner Oberfläche in einem Abstand Noppen oder Einkerbungen als Verschlussmechanismus, in dem ein Teil des Bandes mit den Noppen oder den Einkerbungen versehen ist und die Form der freien Enden des Bandes korrespondierend zu dem Ausnehmungen sind, mittels derer das Band aufnehmbar und mithilfe der Noppen oder Einkerbungen fest arretierbar und wieder lösbar ist.

Dabei geht das Band vom Steg her in Richtung seines freien Endes hin in einen verschmälerten Teil über, wobei die Breite des verschmälerten Teils der Breite der Ausnehmung entspricht und dadurch in die Ausnehmung korrespondierend aufnehmbar ist.

In der zweiten Ausgestaltung (mit zwei Bändern) geht jedes der zwei Bänder vom Steg her in Richtung seines freien Endes hin in einen verschmälerten Teil über, wobei die zwei verschmälerten Teile in ihrer Lage entgegengesetzt und nicht spiegelbildlich sind, und die Breite der zwei verschmälerten Teile der Breite der Ausnehmungen entspricht und dadurch in diese Ausnehmungen korrespondierend aufnehmbar sind, ohne dass sich die zwei Bänder gegenseitig räumlich behindern.

Im aufgenommenen Zustand des Bandes oder der Bänder sind eine oder zwei verstellbare Ösen zur Aufnahme von einem oder zwei Fingern vorausgebildet, welche durch ein Bewegen des Bandes oder der Bänder durch die Ausnehmung oder die Ausnehmungen in ihrem Durchmesser veränderbar sind.

Im Rahmen der Erfindung liegt auch, dass die Orthese mit einer Öse mit einer Fingerauflage versehen ist, um einen Finger längs seines Verlaufes zu fixieren, bzw. dass drei Orthesen mit zwei kleinen Ösen und mittig dazwischenliegend mit einer große Öse mit einer Handballenauflage verbunden sind, um mit der Handballenauflage den Daumen und den keinen Finger einer Hand zu fixieren.

Im Rahmen der Erfindung liegt auch noch, dass in einer weiteren Ausgestaltung ein Relative Motion Splint mit brillenartiger Form bereitgestellt wird, welcher ohne Band oder Riemen sowie ohne Ausnehmung oder Lasche gefertigt ist, in dem sich die beiden dichotomen Enden des Stegs formschlüssig und fest verbunden gegenüber stehen, so dass an Stelle der zangenartig-runde Form eine Ringform ausgebildet ist, welche patientenspezifisch angefertigt ist.

Im Rahmen der Erfindung liegt des Weiteren auch noch, dass, insbesondere bei langwierigen Behandlungen (mit oder ohne morphologische Veränderungen der Finger des Patienten), die Teile der Fingerorthese im Baukastenprinzip zusammenfüg- und austauschbar sind, was zu einer weiteren Kostenminimierung führt.

Die Herstellung der Fingerorthese erfolgt, in dem in einem ersten Teilschritt die patientenspezifischen Daten eines oder mehrerer Patientenfinger durch ein optisches Scanverfahren gemäß dem Stand der Technik ermittelt werden,

Die ermittelten patientenspezifischen Daten werden in einem zweiten Schritt zur Steuerung und Regelung eines an sich bekannten Spritzgussverfahrens oder zur Steuerung und Regelung eines an sich bekannten 3D-Druckverfahrens mit den patientenspezifischen Daten des Stegs und Daten für die Position der Ausnehmungen oder Laschen am Steg eingesetzt, um den Steg aus Kunststoffmaterial zu drucken und dabei die Ausnehmungen oder Laschen zu generieren.

Die Ausnehmungen oder Laschen werden abschließend in einem dritten Schritt mit dem Band oder Riemen aus elastischem, aber zugbeständigem Kunststoff oder Gummi versehen, so dass verstellbare Ösen zur Aufnahme eines oder mehrerer Patientenfinger vorausgebildet sind und die Fingerorthese einsatzbereit ist.

Im Rahmen der Erfindung liegt auch, dass bei der Herstellung des Stegs dem Grundstoffgranulat für den Kunststoff bei der 3-D Drucktechnik spezifischen Zuschlagstoffen, wie insbesondere physiologisch / medizinisch wirkende Stoffen oder auch Farbstoffen hinzugemischt werden können.

Dabei verfügt der Kunstsoff über folgende allgemeine Eigenschaften:
- flexibel, d.h. je nach Orthesenkomponente und physiologisch - medizinischer Wirkung auf die Finger (Fingerführung, Bandmaterial, Steg) härter oder weicherer einstellbarer Kunststoff (dies ist bspw. über das 3D-Druckverfahren und über die Auswahl aus bekannten Grundstoffgranulat einstellbar)
- robust, langlebig,
- leicht,
- Wasser-, Schmutz und Temperatur- unempfindlich,
- sterilisierbar (bspw. wenn die Fingerorthese im OP angelegt wird), und
- optisch ansprechend, wobei eine Farbauswahl möglich ist (dies ist durch zumischen von Farbstoffen beim 3D-Druck einstellbar).

Weiterhin verfügt der Kunstsoff über folgende mechanische und modulare Eigenschaften:
- multifunktionale Einsatzmöglichkeiten - Abdeckung mehrerer Indikationen, Erkrankungen und Verletzungen (dies ist durch den patientenspezifischen 3D-Druck möglich),
- Versorgung von einzelnen oder mehreren Fingern,
- einfach zu installieren (sowohl für den Patienten als auch für den Therapeuten),
- bequem, mit hohem Tragekompfort für den Patienten,
- nicht zu glatt (rutscht somit nicht),
- flexibel, trotzdem stützend und ggf. sofern gewünscht auch leicht dehnbar (dies ist durch den patientenspezifischen 3D-Druck möglich)
- abgerundete Kanten ohne spitze Erhebungen bzw. "Nasen" (Vermeidung von Verletzungen, Druckstellen) und
- Wandstarke ausreichend stützend, nicht zu dick.

Des Weiteren verfügt der zur Herstellung der Fingerorthese verwendete Kunstsoff über folgende physiologisch - therapeutische Eigenschaften:
- stabilisierend,
- stutzend, entlastend,
- funktionswiederherstellend,
- lagernd,
- heilungsfordernd und
- mobilisierend.

Darüber hinaus liegt auch im Rahmen der Erfindung, dass aus Kostengründen vorgesehen ist, die einzelnen Komponenten der Fingerorthese in unterschiedlichen Standartgrößen im thermoplastischen Spritzguss zu fertigen.

Die teurere Variante des Herstellungsverfahrens mit einem Scan der Hand des Patienten zur Erzeugung eines 3D- Modelles und nachfolgendem 3D- Druck ist insbesondere dann anzuwenden, wenn eine Sonderlösung oder die anatomischen Gegebenheiten dies erfordern.

Verbunden mit den Eigenschaften der bereitgestellten Fingerorthese sind die folgenden Vorteile:
Die technische Lösung ermöglicht gegenüber dem Stand der Technik eine effektivere und kostengünstigere, insbesondere individuelle Bereitstellung von patientenspezifischen Fingerorthesen, die als:
- relative Motion Splint für die Extension des MCP/Mittelfinger - Grundgelenk
- Ringbandschutzschiene bei Ringbanderkrankungen und Verletzungen
- weichen Ringbandschutzschiene
- Block- Schiene bei *Tendovaginitis stenosans* (Schnappfinger)
- doppelte Ringbandschutzschiene oder
- Achterschlaufe bzw. Kombinationsschiene für mehrere Finger
eingesetzt werden können, wobei ein guter Halt /Sitz bei gleichzeitig mehrfacher Lösung und Wiederanlegung der Orthese und ein verbesserter Tragkomfort bei einfacher Handhabbarkeit für den Patienten erzielt wird.

Des Weiteren besteht die Möglichkeiten, bei der bereitgestellten Fingerprothese eine Optimierung und Variation in einer Art Baukastensystem vorzunehmen, was folgende Vorteile mit sich bringt:
- Versorgung von allen sowie multiplen Fingern ist gewährleistet durch einen Schienenaufbau in dem beispielsweisen Baukastenprinzip:
   → Fingerringe und Fingersteg separieren zur individuellen Modulation
   → Konfiguration verschiedener Steglangen
   → Klicksystem: Komponenten (Steg/Fingerringe) verbinden, was dazu führt, dass
      - einer bzw. mehrere Finger können in MCP- Extension oder Flexion gebracht werden
      - das Fingerringsystem zur Versorgung von multiplen Indikationen, Erkrankungen und Verletzungen einzeln anwendbar ist

Die Erfindung wird nachstehend an Hand der Ausführungsbeispiele und der Figuren näher erläutert, ohne auf diese beschränkt zu werden.

Dazu zeigen:
- Fig. 1:: eine schematische Darstellung einer Ausführungsform gemäß dem Stand der Technik,
- Fig. 2a:: eine schematische 3D-Darstellung einer ersten Ausführungsform der erfindungsgemäßen Fingerorthese (mit einer Mulde zur Auflage von einem Finger),
- Fig. 2b:: eine seitliche Darstellung der Ausführungsform gemäß Fig. 1

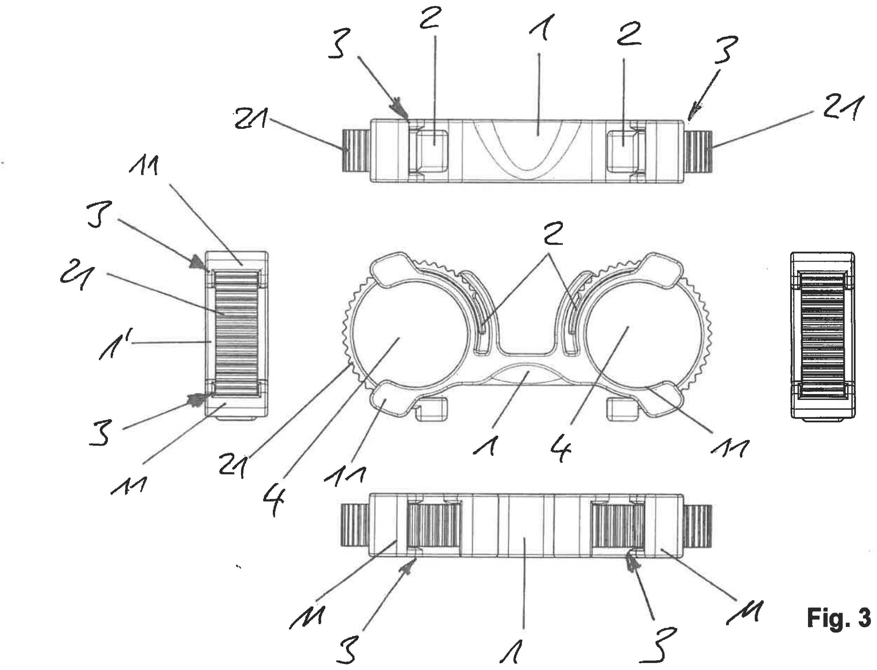

- Fig. 3:: eine Darstellung der Ausführungsform gemäß Fig. 1 mit sämtlichen Seitenansichten,
- Fig. 4:: eine schematische Darstellung einer zweiten Ausführungsform der erfindungsgemäßen Fingerorthese (mit zwei Mulden zur Auflage von zwei Fingern),

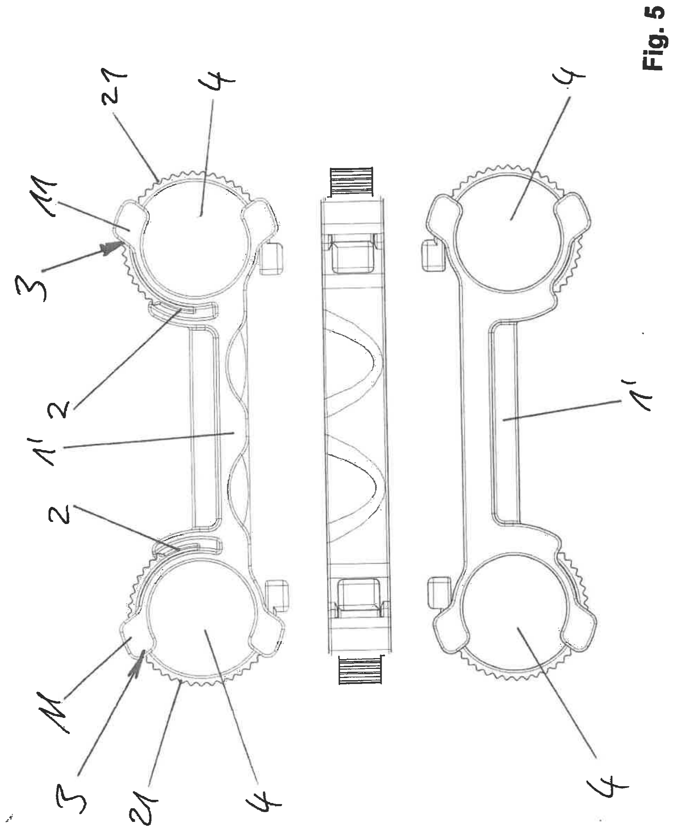

- Fig. 5:: eine Darstellung der Ausführungsform gemäß Fig. 4 in drei verschiedenen Seitenansichten,
- Fig. 6:: eine schematische Darstellung einer dritten Ausführungsform der erfindungsgemäßen Fingerorthese in geöffneter Form in drei Ansichten,
- Fig. 7:: eine Darstellung der Ausführungsform gemäß Fig. 6 in geschlossener Form mit zwei Ösen (Doppelschlaufe für zwei Finger),
- Fig. 8:: eine schematische Darstellung einer vierten Ausführungsform der erfindungsgemäßen Fingerorthese in geöffneter Form in drei Ansichten,
- Fig. 9:: eine Darstellung der Ausführungsform gemäß Fig. 8 in geschlossener Form mit einer Öse (Einfachschlaufe für einen Finger),

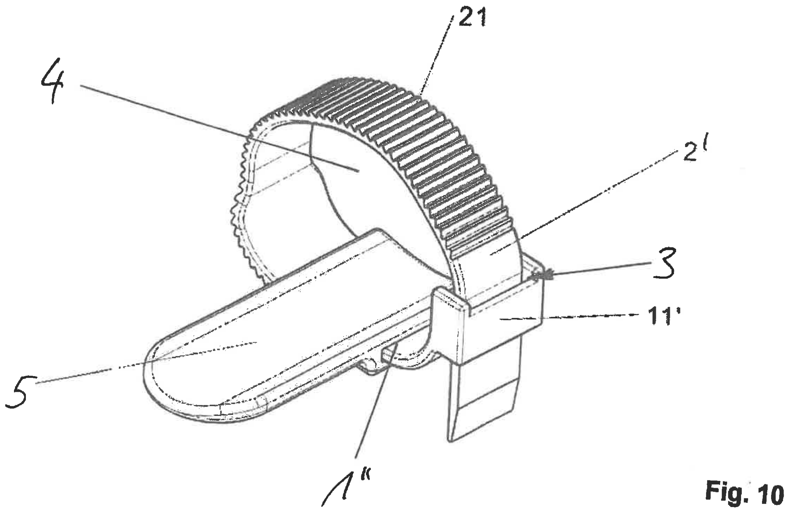

- Fig.10:: eine schematische Darstellung einer fünften Ausführungsform der erfindungsgemäßen Fingerorthese in geschlossener Form mit einer Öse gemäß Fig. 9 mit einer Fingerauflage,

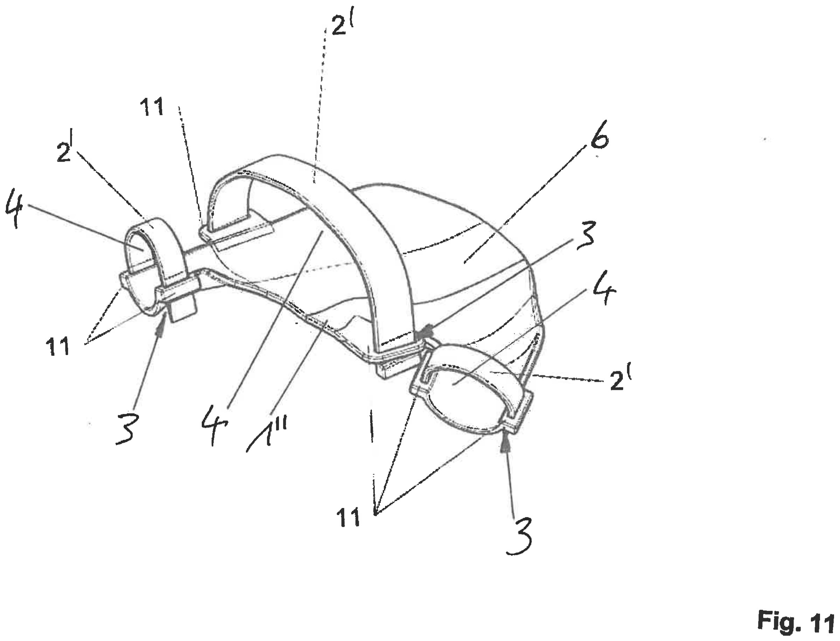

- Fig.11:: eine schematische Darstellung einer sechsten Ausführungsform der erfindungsgemäßen Fingerorthese in geschlossener Form mit zwei kleinen Ösen gemäß Fig. 9, einer dazwischenliegenden großen Öse mit einer Handballenauflage sowie
- Fig.12:: eine schematische Darstellung der Ausführungsform gemäß Fig. 11 im angelegten Zustand an einer Hand mit Handballenauflage und Fixierung von Daumen und keinem Finger.

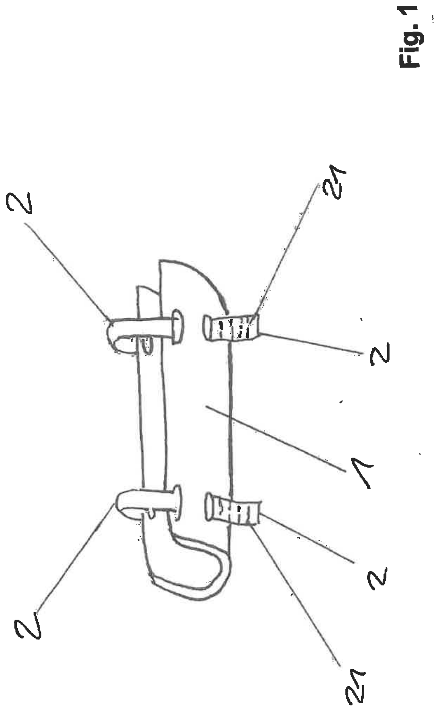

Die Fig. 1 zeigt eine Fingerorthese gemäß dem Stand der Technik. Diese Orthese umfasst eine an der Ober- oder Unterseite eines Fingers anbringbare, zu dem Finger längs verlaufende Schiene (1), deren Länge der Länge des Fingers entspricht sowie zwei Bänder (2) mit einem Verschlussmechanismus (3), wobei die Schiene (1) aus Kunststoff ist und im Bereich ihrer freien Enden über jeweils zwei, sich gegenüber liegende Ausnehmung (11) verfügt, und die Bänder (2) aus elastischem, zugbeständigem Kunststoff oder Gummi bestehen und einen Verschlussmechanismus (3) ausbilden, in dem die Ausnehmungen (11) in ihren Form korrespondierend zu den Bändern (2) sind und mittels derer die Bänder (2) aufgenommen werden, in dem je ein Band (2) jeweils durch die zwei gegenüber liegenden Ausnehmung (11) eines freien Endes der Schiene (1) verläuft sowie fest arretier- und wieder lösbar ist, in dem das Band (2) an einem seiner freien Enden gegenüber einer der Ausnehmungen (11) geblockt ist.

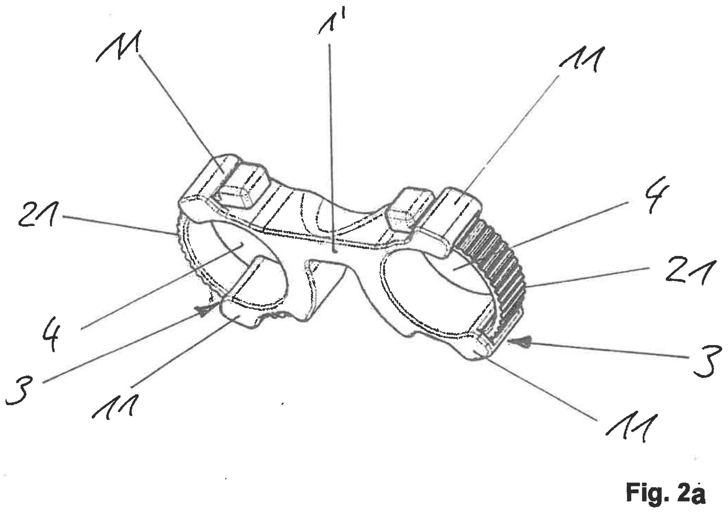

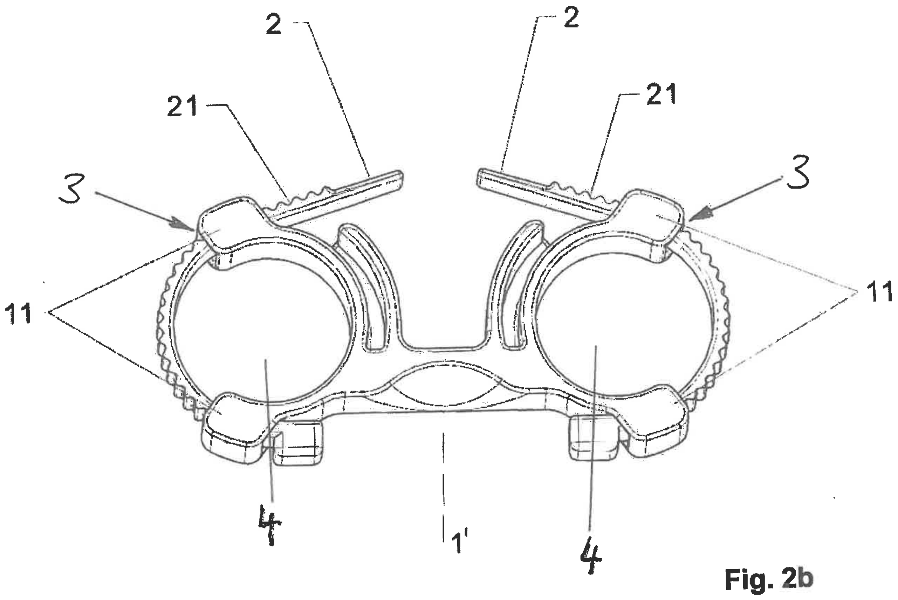

Ausgehend von diesem Stand der Technik werden bspw. drei Varianten einer neuen Fingerorthese bereitgestellt, welche in den Figuren 2a, 2 b, sowie 4 und 7 gezeigt sind.

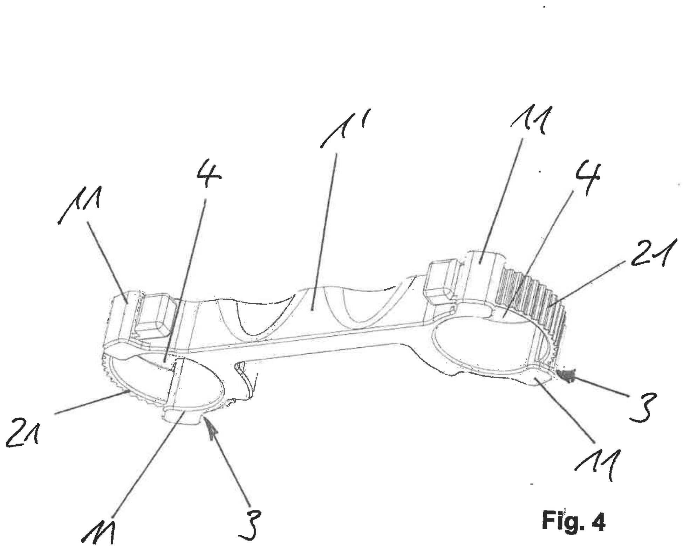

Die in den Fig. 2a und 2b gezeigte erste Ausführungsform und die in der Fig. 4 dargestellte zweite Ausführungsform der Fingerorthese umfasst eine, mindestens an einem Finger anbringbare, gegenüber den Fingern längs verlaufende Schiene (1), sowie zwei Bänder (2) mit einen Verschlussmechanismus (3), welche gegenüber dem Stand der Technik wie folgt abgewandelt sind:
Die Breite der Schiene (1) [welche der Länge der Schiene (1) gemäß Fig. 1 entspricht] ist kürzer als ein Fingerglied und bildet einen Steg (1') aus, der zwischen zwei Fingern anordenbar ist sowie länger als zwei Fingerbreiten ist.

Dieser Steg (1') besteht aus Kunststoff und geht im Bereich seiner beiden freien Enden dichotom in jeweils zwei Enden über, die jeweils zwei, sich gegenüberliegende Ausnehmung oder Laschen (11) aufweisen, wobei die Bänder (2) aus elastischem, zugbeständigem Kunststoff oder Gummi bestehen und den Verschlussmechanismus (3) tragen, in dem die Bänder (2) auf einer Seite der Oberfläche mit den Noppen oder den Einkerbungen (21) versehen sind und die Form der freien Enden der Bänder (2) korrespondierend zu dem Ausnehmungen oder Laschen (11) sind, mittels derer sie aufnehmbar und mithilfe der Noppen oder Einkerbungen (21) fest arretierbar und wieder lösbar sind. Dabei verläuft je ein Band (2) jeweils durch die gegenüberliegenden Ausnehmung oder Laschen (11) eines der zwei freien Enden des Stegs (1') und ist fest arretier- und wieder lösbar, in dem es an einem seiner freien Enden gegenüber einer der Ausnehmungen oder Laschen (11) geblockt ist,

Dadurch werden im aufgenommenen Zustand jedes Bandes (2) verstellbare Ösen (4) zur Aufnahme eines oder mehrerer Finger vorausgebildet, welche durch ein Bewegen der Bänder (2) in den Ausnehmungen oder Laschen (11) in ihrem Durchmesser veränderbar sind.

Die Ausführungsform gemäß der Fig. 2 unterscheidet sich dabei von der Ausführungsform gemäß der Fig. 4 insbesondere dadurch, dass der Steg (1') in Fig. 2 etwas schmaler (und mit einer Mulde zur Auflage von einem Finger) ausgeführt ist, als der Steg (1') in der Fig. 4 (mit zwei Mulden zur Auflage von zwei Fingern).

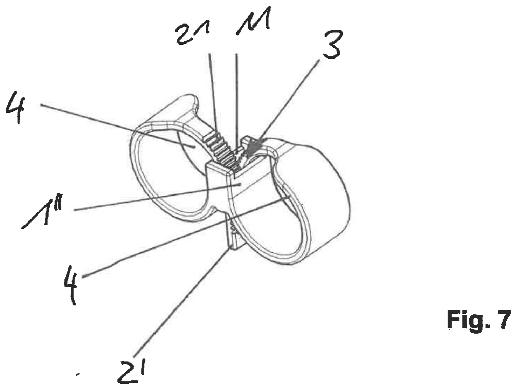

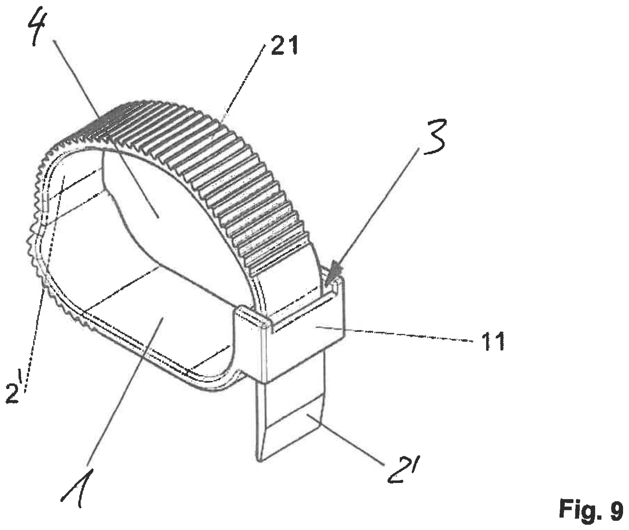

In Fig. 7 (Doppelschlaufe für zwei Finger) und Fig. 9 (Einfachschlaufe für einen Finger) sind die Ausführungsformen zweier weiterer Varianten der Fingerorthese mit zwei Ösen bzw. einer Öse für die Fingeraufnahme dargestellt.

Diese beiden Ausführungsformen der Fingerorthese umfassen eine, an einem oder zwei Fingern anbringbare, gegenüber den Fingern längs verlaufende Schiene (1), sowie zwei Bänder (2) mit einen Verschlussmechanismus (3), wobei die Breite der Schiene (1) kürzer als ein Fingerglied ist und einen Steg (1") ausbildet, der länger als zwei Fingerbreiten ist und der Steg (1") zwischen zwei Fingern längs verlaufend zu den Fingern anordenbar ist und ein- oder beidseitig in ein Band (2') mit einem Verschlussmechanismus (3) übergeht.

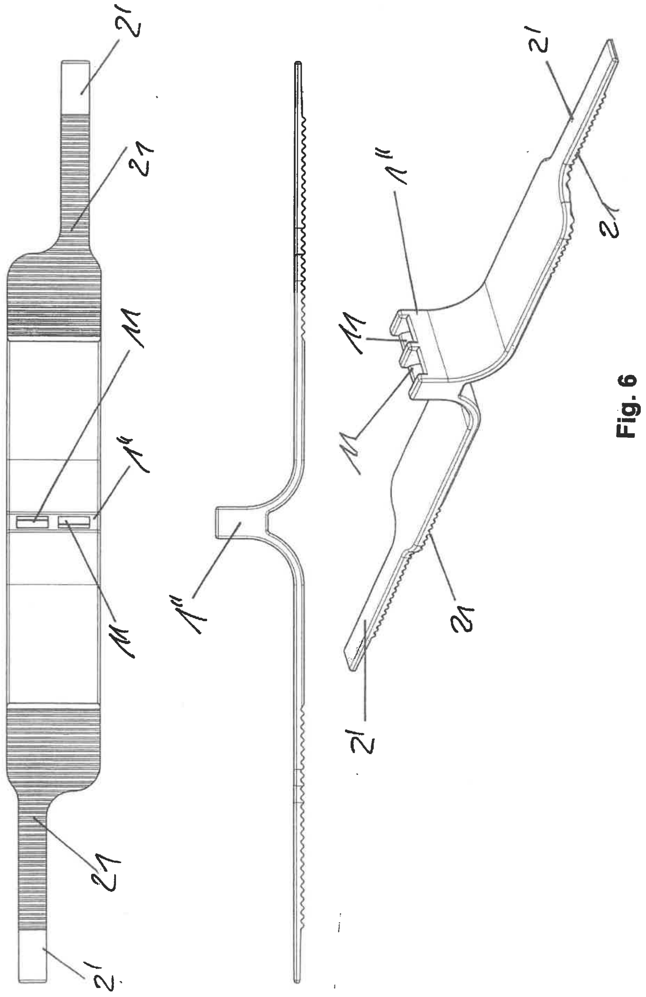

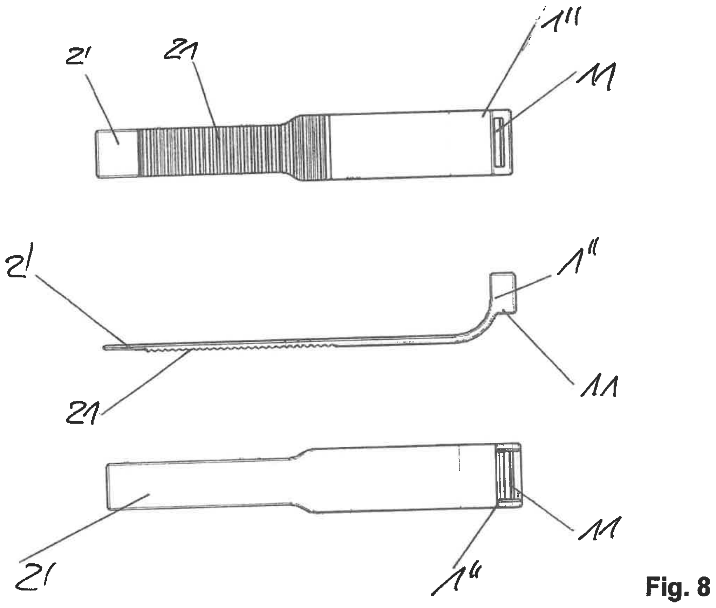

Dabei sind der Steg (1") und das Band (2') einstückig und bestehen aus Kunststoff, wobei der Steg (1") mittig angeordnet ist und über eine Ausnehmung [siehe Fig. 8 und 9 bei der Einfachschlaufe für eine Öse zur Aufnahme von einem Finger] oder zwei Ausnehmungen (11) [siehe Fig. 6 und 7 bei der Doppelschlaufe für zwei Ösen zur Aufnahme von zwei Fingern] verfügt sowie gegenüber dem Band (2') in seiner Konsistenz fester und härter ist.

Dabei ist das Band (2') elastisch sowie zugbeständig und trägt über zumindest einen Teil der Gesamtlänge zumindest auf einer Seite auf seiner Oberfläche in einem Abstand Noppen oder Einkerbungen (21') als Verschlussmechanismus (3), in dem ein Teil des Bandes (2') mit den Noppen oder den Einkerbungen (21) versehen ist und die Form der freien Enden des Bandes (2') korrespondierend zu dem Ausnehmungen (11) sind, mittels derer das Band (2') aufnehmbar und mithilfe der Noppen oder Einkerbungen (21) fest arretierbar und wieder lösbar ist.

Für die Variante gemäß Fig. 9 [Einfachschlaufe für einen Finger mit einem Band (2')] ist am Steg (1") geht dieses Band (2) vom Steg (1) her in Richtung seines freien Endes hin in einen verschmälerten Teil über, wobei die Breite des verschmälerten Teils der Breite der Ausnehmungen (11) entspricht und dadurch in die Ausnehmung (11) korrespondierend aufnehmbar ist.

Für die Variante gemäß Fig. 7 [Doppelschlaufe für zwei Finger mit zwei Bändern (2') links sowie rechts am Steg (1")] geht jedes der zwei Bänder (2') vom Steg (1") her in Richtung seines freien Endes hin in einen verschmälerten Teil über, wobei die zwei verschmälerten Teile in ihrer Lage entgegengesetzt und nicht spiegelbildlich sind, und die Breite der zwei verschmälerten Teile der Breite der Ausnehmungen (11) entspricht und dadurch in diese Ausnehmungen (11) korrespondierend aufnehmbar sind, ohne dass sich die zwei Bänder (2') gegenseitig räumlich behindern. Verdeutlicht wird diese Ausgestaltung in der Darstellung der Fig. 6.

Im aufgenommenen Zustand des Bandes (2') [siehe Fig. 9] oder der Bänder (2') [siehe Fig. 7] sind eine oder zwei verstellbare Ösen (4) zur Aufnahme von einem oder zwei Fingern vorausgebildet, welche durch ein Bewegen des Bandes (2') oder der Bänder (2) durch die Ausnehmung (11) oder die Ausnehmungen (11) in ihrem Durchmesser veränderbar sind.

Vorteilhaft bei allen drei Varianten ist, dass:
- die Noppen oder Einkerbungen (21) auf der Außenseite des, die Öse (4) bildenden Bandes (2; 2') angeordnet sind,
- der Abstand der Noppen oder Einkerbungen (21) zueinander regelmäßig ist,
- der Steg (1'; 1") und das Band (2; 2') anhand patientenspezifischer Computertomographiedaten per 3D-Druck- oder Spritzgussverfahren aus Kunststoffpulver hergestellt sind,
- der Kunstsoff mit Wirkstoffen versehen ist, welche aus dem Kunststoff in den Finger diffundieren können
- und insbesondere bei den Ausführungsformen gemäß Fig. 2 und Fig. 4, dass der Steg (1'oder 1") an seinen beiden Enden dichotom in je eine zangenartig-runde Form übergeht, wobei die Ausnehmungen oder Laschen (11) sich gegenüber liegen und mit den Bändern (2 oder 2') die Ösen (4) bilden, so dass ein Relative Motion Splint mit brillenartiger Form ausgebildet ist.

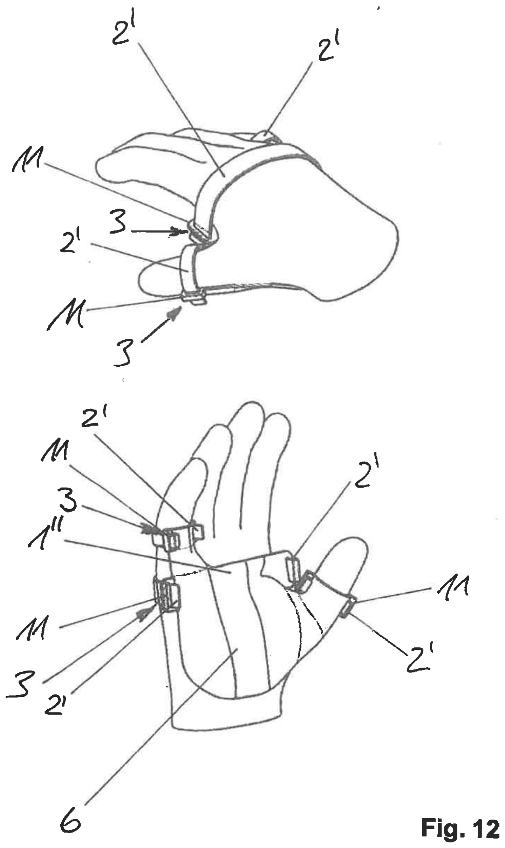

Im Rahmen der Erfindung liegt auch noch bei einer fünften Ausführungsform der erfindungsgemäßen Fingerorthese die Orthese gemäß Fig. 9 (geschlossener Form mit einer Öse) mit einer Fingerauflage zu versehen, bzw. in einer sechsten Ausführungsform der erfindungsgemäßen Fingerorthese zwei kleinen Ösen gemäß Fig. 9 und eine dazwischenliegende große Öse Fig. 9 mit einer Handballenauflage zu versehen, um mit der Handballenauflage den Daumen und den keinen Finger einer Hand zu fixieren (siehe Fig. 12).

Die Herstellung der Fingerorthese erfolgt somit in dem in einem ersten Teilschritt die patientenspezifischen Daten eines oder mehrerer Patientenfinger durch ein optisches Scanverfahren gemäß dem Stand der Technik ermittelt werden,

Die ermittelten patientenspezifischen Daten werden in einem zweiten Schritt zur Steuerung und Regelung eines an sich bekannten 3D-Druck- oder Spritzgussverfahrens mit den patientenspezifischen Daten des Stegs (1) und Daten für die Position der Ausnehmungen oder Laschen (11) am Steg (1) eingesetzt, um den Steg (1'; 1") aus Kunststoffmaterial zu drucken und dabei die Ausnehmungen oder Laschen (11) zu generieren.

Die Ausnehmungen oder Laschen (11) werden, wenn das Band (2') oder die Bänder (2; 2') nicht einstückiger Bestandteil des Stegs (1'; 1) sind, abschließend in einem dritten Schritt mit dem Band (2') oder den Bändern (2, 2') aus elastischem, aber zugbeständigem Kunststoff oder Gummi versehen, so dass verstellbare die Ösen (4) zur Aufnahme eines oder mehrerer Patientenfinger vorausgebildet sind und die Fingerorthese einsatzbereit ist.

Im Rahmen der Erfindung liegt auch, dass bei der Herstellung des Stegs (1'; 1") dem Grundstoffgranulat für den Kunststoff bei dem 3-D Druck- oder der Spritzgussverfahren spezifische Zuschlagstoffen, wie insbesondere physiologisch / medizinisch wirkende Stoffen oder auch Farbstoffen, hinzugemischt werden können.

Mit der bspw. in Fig. 2 gezeigten Fingerorthese in Form einer Relative Motion Splint besteht der Möglichkeit der Versorgung von einem Finger, (bspw. Mittel- oder Ringfinger) oder der Versorgung von zwei Fingern (bspw. für die Lagerung des MCP von Ring- und Mittelfinger in Streckung) mittels des mehrfach verstell- und verwendbaren Verstellmechanismus mit den flexible hinsichtlich der Länge einstellbaren Bändern (2) (zur Fixation für verschiedene Fingerumfange), wobei der flexible Kunststoff des Stegs (1) genügend Stabilität im Bereich Fingerkerbe aufweist.

Im Rahmen der Erfindung liegt auch, dass die Fingerorthese in Form eines individuell im 3D-Druck- oder Spritzgussverfahren hergestellten, flexiblen Relative Motion Splints mit einer "Brillenartige" Form bereitgestellt wird, welcher ohne Band (2; 2') (d.h. ohne Einstellmöglichkeiten) aus elastischem Kunststoff mit einer Kerbe als Fingerführung und zwei Einschlupflöchern für die Finger [an Stelle der bspw. in den Figuren 2 und 4 ersichtlichen zangenartig-runden dichotomen Enden des Stegs (1'; 1") mit den Ösen (4)] ausgeführt ist (in dem sich die beiden dichotomen Enden des Stegs (1) formschlüssig und fest verbunden gegenüber stehen) und die Möglichkeit der patientenspezifischen Versorgung von jeweils einem Finger (bspw. Mittel- oder Ringfinger) bietet (in den Figuren nicht dargestellt).

Im Rahmen der Erfindung liegt auch, dass in einer weiteren Ausführungsform ein Relative Motion Splint mit brillenartiger Form bereitgestellt wird, bei welcher, wie in bspw. in der Fig. 7 gezeigt, eine bereitgestellte Fingerorthese in Form einer individuell anpassbaren, flexiblen "Achterschlaufe" (= Kombinationsschiene für mehrere Finger nebeneinander liegende Finger, bspw. Zeige- und Mittelfinger) ausgeführt ist, bei welcher der Steg (1'; 1") so weit verkürzt ist, dass seine beiden dichotomen Enden mit dem mehrfach verstell- und verwendbaren Verschlusssystem, das die einstellbaren Fingerschlaufen ausbildet, unmittelbar nebeneinander, aber dennoch voneinander gering beabstandet angeordnet sind, so dass die Achterschlaufe an verschiedenen Stellen der Finger nutzbar und mit mehreren Fingern kombinierbar ist, was dazu führt, dass die Achterschlaufe in verschiedenen Bereichen der Fingerlange (auch doppelt) anwendbar ist.

Im Rahmen der Erfindung liegt des Weiteren auch, dass, insbesondere bei langwierigen Behandlungen (mit oder ohne morphologische Veränderungen der Finger des Patienten), die Teile der Fingerorthese im Baukastenprinzip zusammenfüg- und austauschbar sind, was zu einer weiteren Kostenminimierung führt.

Die Verwendung der zuvor stehend beschriebenen Fingerorthese ist somit sehr vielfältig, bspw. als Frühmobilisations- Blockungs- oder Entlastungsschienen der Finger eines Patienten, oder als Relative Motion Splint.

Wesentliche Anwendungsmöglichkeiten sind als
- Relative Motion Splint (1 Finger), bei dem Mittel- oder Zeigefinger gestützt wird,
- Relative Motion Splint (2 Finger), bei dem Mittel- und Ringfinger gestützt werden,
- Ringbandschutz (1 Finger), bei dem ein Finger durch Schlaufe stabilisiert wird,
- Ringbandschutz (2 Finger), bei dem ein Finger durch einen danebenliegenden Finger stabilisiert wird,
- Ringbandschutz mit verlängerter Auflage, bei dem bei einem Schnappfinger ein Schnallen des Fingers nach unten verhindert wird
   oder
- als Schiene für alle Finger als Auflage zur Stabilisierung von Daumen und jedem anderen Finger.

Der Vorteil sämtlicher Ausführungsformen der bereitgestellten Fingerorthese besteht in ihren flexiblen, multifunktionalen und modularen Eigenschaften, welche basierend auf den erfassen speziellen Daten eines Patenten mithilfe der 3D-Technik patientenspezifisch, preiswert und gleichzeitig hohen und individuell anpassbaren Tragekomfort herstellbar ist sowie robust, langlebig, leicht und optisch ansprechend ist.

Des Weiteren ermöglicht die bereitgestellte Fingerorthese die Versorgung von einzelnen oder mehreren Fingern, wobei sie für den Patient und Therapeut einfach zu installieren und nach dem Anlegen für den Patienten bequem ist, wobei sie nicht rutscht und gleichzeitig flexibel, stützend und leicht dehnbar ist.

Alle in der Beschreibung, den Ausführungsbeispielen und den nachfolgenden Ansprüchen dargestellten Merkmale können sowohl einzeln als auch in beliebiger Kombination miteinander erfindungswesentlich sein.

### Bezugszeichen

- 1 -: Schiene
- 1'; 1"-: Steg
- 11 -: Ausnehmung oder Lasche
- 2; 2' -: Band
- 21 -: Noppen oder Einkerbung
- 3 -: Verschlussmechanismus
- 4 -: Öse
- 5 -: Fingerauflage am Steg
- 6 -: Handballenauflage, dem Steg entspringend mit drei Ösen

## Patentansprüche

1. Fingerorthese umfassend eine, mindestens an einem Finger anbringbare, gegenüber den Fingern längs verlaufende Schiene (1), sowie zwei Bänder (2) mit einen Verschlussmechanismus (3), **dadurch gekennzeichnet, dass**
- die Breite der Schiene (1) kürzer als ein Fingerglied ist und einen Steg (1') ausbildet, der zwischen zwei Fingern anordenbar ist sowie länger als zwei Fingerbreiten ist,
- der Steg (1') aus Kunststoff besteht und im Bereich seiner beiden freien Enden dichotom in jeweils zwei Enden übergeht, die jeweils zwei, sich gegenüberliegende Ausnehmung oder Laschen (11) aufweisen,
- die Bänder (2) aus elastischem, zugbeständigem Kunststoff oder Gummi bestehen und den Verschlussmechanismus (3) tragen, in dem die Bänder (2) auf einer Seite der Oberfläche mit Noppen oder Einkerbungen (21) versehen sind und die Form der freien Enden der Bänder (2) korrespondierend zu den Ausnehmungen oder Laschen (11) sind, mittels derer sie aufnehmbar und mithilfe der Noppen oder Einkerbungen (21) fest arretierbar und wieder lösbar sind,
wobei je ein Band (2) jeweils durch die gegenüberliegenden Ausnehmung oder Laschen (11) eines der zwei freien Enden des Stegs (1') verläuft sowie fest arretier- und wieder lösbar ist, in dem es an einem seiner freien Enden gegenüber einer der Ausnehmungen oder Laschen (11) geblockt ist,
so dass im aufgenommenen Zustand jedes Bandes (2) verstellbare Ösen (4) zur Aufnahme eines oder mehrerer Finger vorausgebildet sind, welche durch ein Bewegen der Bänder (2) in den Ausnehmungen oder Laschen (11) in ihrem Durchmesser veränderbar sind.

2. Fingerorthese umfassend eine, an einem oder zwei Fingern anbringbare, gegenüber den Fingern längs verlaufende Schiene (1), sowie ein Band (2) oder zwei Bänder (2) mit einen Verschlussmechanismus (3), **dadurch gekennzeichnet, dass**
- die Breite der Schiene (1) kürzer als ein Fingerglied ist und einen Steg (1") ausbildet, der länger als zwei Fingerbreiten ist,
- dass der Steg (1") zwischen zwei Fingern längs verlaufend zu den Fingern anordenbar ist und ein- oder beidseitig in ein Band (2') mit einem Verschlussmechanismus (3) übergeht,
- der Steg (1") und das Band (2') einstückig sind und aus Kunststoff bestehen,
- der Steg (1") mittig angeordnet über ein oder zwei Ausnehmungen (11) verfügt sowie gegenüber dem Band (2') in seiner Konsistenz fester und härter ist,
- das Band (2') elastisch sowie zugbeständig ist und über zumindest einen Teil der Gesamtlänge zumindest auf einer Seite auf seiner Oberfläche in einem Abstand Noppen oder Einkerbungen (21') als Verschlussmechanismus (3) trägt, in dem ein Teil des Bandes (2') mit den Noppen oder den Einkerbungen (21) versehen ist und die Form der freien Enden des Bandes (2') korrespondierend zu den Ausnehmungen (11) sind, mittels derer das Band (2') aufnehmbar und mithilfe der Noppen oder Einkerbungen (21) fest arretierbar und wieder lösbar ist, wobei
- bei einem Band (2') am Steg (1") dieses Band (2) vom Steg (1) her in Richtung seines freien Endes hin in einen verschmälerten Teil übergeht und die Breite des verschmälerten Teils der Breite der Ausnehmungen (11) entspricht und dadurch in die Ausnehmung (11) korrespondierend aufnehmbar ist,
oder
- bei je einem Band (2') links sowie rechts am Steg (1") jedes der zwei Bänder (2') vom Steg (1") her in Richtung seines freien Endes hin in einen verschmälerten Teil übergeht, wobei die zwei verschmälerten Teile in ihrer Lage entgegengesetzt und nicht spiegelbildlich sind, und die Breite der zwei verschmälerten Teile der Breite der Ausnehmungen (11) entspricht und dadurch in diese Ausnehmungen (11) korrespondierend aufnehmbar sind, ohne dass sich die zwei Bänder (2') gegenseitig räumlich behindern,
so dass im aufgenommenen Zustand des Bandes (2') oder der Bänder (2') eine oder zwei verstellbare Ösen (4) zur Aufnahme von einem oder zwei Fingern vorausgebildet sind, welche durch ein Bewegen des Bandes (2') oder der Bänder (2) durch die Ausnehmung (11) oder die Ausnehmungen (11) in ihrem Durchmesser veränderbar sind.

3. Fingerorthese gemäß Anspruch 1 oder 2, **dadurch gekennzeichnet, dass** die Noppen oder Einkerbungen (21) auf der Außenseite des, die Öse (4) bildenden Bandes (2; 2') angeordnet sind.

4. Fingerorthese gemäß einem oder mehrerer der Ansprüche 1, 2 oder 3, **dadurch gekennzeichnet, dass** der Abstand der Noppen oder Einkerbungen (21) zueinander regelmäßig ist.

5. Fingerorthese gemäß Anspruch 3 und 4, **dadurch gekennzeichnet, dass** der Steg (1'; 1") und das Band (2; 2') anhand patientenspezifischer Computertomographiedaten per 3D-Druck- oder Spritzgussverfahren aus Kunststoffpulver hergestellt sind.

6. Fingerorthese gemäß einem oder mehrerer der Ansprüche 1 bis 5, **dadurch gekennzeichnet, dass** der Kunstsoff mit Wirkstoffen versehen ist, welche aus dem Kunststoff in den Finger diffundieren können.

7. Fingerorthese gemäß einem oder mehrerer der Ansprüche 1, 3, 4, 5 oder 6, **dadurch gekennzeichnet, dass** der Steg (1'; 1") an seinen beiden Enden dichotom in je eine zangenartig-runde Form übergeht, wobei die Ausnehmungen oder Laschen (11) sich gegenüber liegen und mit den Bändern (2 oder 2') die Ösen (4) bilden, so dass ein Relative Motion Splint mit brillenartiger Form ausgebildet ist.

8. Verfahren zur Herstellung einer Fingerorthese gemäß einem oder mehrerer der Ansprüche 1 bis 7 bei dem patientenspezifische Daten eines oder mehrerer Patientenfinger durch ein optisches Scanverfahren ermittelt werden, die ermittelten patientenspezifischen Daten zur Steuerung und Regelung eines 3D-Druck- oder Spritzguss- verfahrens mit patientenspezifischen Daten des Stegs (1'; 1") und Daten für die Position der Ausnehmungen oder Laschen (11) am Steg (1'; 1") eingesetzt werden, um den Steg (1'; 1") und das Band (2') aus Kunststoffmaterial zu drucken, oder die Ausnehmungen oder Laschen (11) mit dem Band (2') oder die Bänder (2 oder 2') zu versehen, so dass eine verstellbare Ösen (4) oder zwei verstellbare Ösen (4) zur Aufnahme eines oder zwei Patientenfinger vorausgebildet sind.

9. Verwendung einer Fingerorthese gemäß einem oder mehrerer der Ansprüche 1 bis 7 als Frühmobilisations- Blockungs- oder Entlastungsschienen der Finger eines Patienten,

10. Verwendung einer Fingerorthese gemäß einem oder mehrerer der Ansprüche 1 bis 7 als Relative Motion Splint.

## Geänderte Patentansprüche

### Geänderte Patentansprüche gemäss Regel 137(2) EPÜ.

1. Fingerorthese umfassend eine, mindestens an einem Finger anbringbare, gegenüber den Fingern längs verlaufende Schiene (1), sowie mindestens ein Band (2) mit einem Verschlussmechanismus (3), wobei
- die Breite der Schiene (1) kürzer als ein Fingerglied ist und einen Steg (1') ausbildet, der zwischen zwei Fingern anordenbar ist sowie länger als zwei Fingerbreiten ist,
- der Steg (1') aus Kunststoff besteht und im Bereich seiner beiden freien Enden dichotom in jeweils zwei Enden übergeht, die jeweils zwei, sich gegenüberliegende Ausnehmung oder Laschen (11) aufweisen,
- das Band (2) aus elastischem, zugbeständigem Kunststoff oder Gummi bestehen und den Verschlussmechanismus (3) tragen, **dadurch gekennzeichnet, dass** das Band (2) auf einer Seite der Oberfläche mit Noppen oder Einkerbungen (21) versehen sind und die Form des freien Endes des Bandes (2) korrespondierend zu den Ausnehmungen oder Laschen (11) sind, mittels derer es aufnehmbar und mithilfe der Noppen oder Einkerbungen (21) fest arretierbar und wieder lösbar sind,
wobei das Band (2) jeweils durch die gegenüberliegenden Ausnehmung oder Laschen (11) eines der zwei freien Enden des Stegs (1') verläuft sowie fest arretier- und wieder lösbar ist, in dem es an einem seiner freien Enden gegenüber einer der Ausnehmungen oder Laschen (11) geblockt ist,
so dass im aufgenommenen Zustand jedes Bandes (2) verstellbare Ösen (4) zur Aufnahme eines oder mehrerer Finger vorausgebildet sind, welche durch ein Bewegen des Bandes (2) in den Ausnehmungen oder Laschen (11) in ihrem Durchmesser veränderbar sind.

2. Fingerorthese gemäß Anspruch 1, **dadurch gekennzeichnet, dass** die Schiene an einem oder zwei Fingern anbringbar ist und ein Band (2) oder zwei Bänder (2) mit einen Verschlussmechanismus (3) vorgesehen sind, wobei
- die Breite der Schiene (1) kürzer als ein Fingerglied ist und einen Steg (1") ausbildet, der länger als zwei Fingerbreiten ist,
- dass der Steg (1") zwischen zwei Fingern längs verlaufend zu den Fingern anordenbar ist und ein- oder beidseitig in ein Band (2') mit einem Verschlussmechanismus (3) übergeht,
- der Steg (1") und das Band (2') einstückig sind,
- der Steg (1") mittig angeordnet über ein oder zwei Ausnehmungen (11) verfügt sowie gegenüber dem Band (2') in seiner Konsistenz fester und härter ist,
- das Band (2') elastisch sowie zugbeständig ist und über zumindest einen Teil der Gesamtlänge zumindest auf einer Seite auf seiner Oberfläche in einem Abstand Noppen oder Einkerbungen (21') als Verschlussmechanismus (3) trägt, in dem ein Teil des Bandes (2') mit den Noppen oder den Einkerbungen (21) versehen ist und die Form der freien Enden des Bandes (2') korrespondierend zu den Ausnehmungen (11) sind, mittels derer das Band (2') aufnehmbar und mithilfe der Noppen oder Einkerbungen (21) fest arretierbar und wieder lösbar ist, wobei
- bei einem Band (2') am Steg (1") dieses Band (2) vom Steg (1) her in Richtung seines freien Endes hin in einen verschmälerten Teil übergeht und die Breite des verschmälerten Teils der Breite der Ausnehmungen (11) entspricht und dadurch in die Ausnehmung (11) korrespondierend aufnehmbar ist,
so dass im aufgenommenen Zustand des Bandes (2') oder der Bänder (2') eine oder zwei verstellbare Ösen (4) zur Aufnahme von einem oder zwei Fingern vorausgebildet sind, welche durch ein Bewegen des Bandes (2') oder der Bänder (2) durch die Ausnehmung (11) oder die Ausnehmungen (11) in ihrem Durchmesser veränderbar sind.

3. Fingerorthese gemäß Anspruch 2, **dadurch gekennzeichnet, dass** bei je einem Band (2') links sowie rechts am Steg (1") jedes der zwei Bänder (2') vom Steg (1") her in Richtung seines freien Endes hin in einen verschmälerten Teil übergeht, wobei die zwei verschmälerten Teile in ihrer Lage entgegengesetzt und nicht spiegelbildlich sind, und die Breite der zwei verschmälerten Teile der Breite der Ausnehmungen (11) entspricht und dadurch in diese Ausnehmungen (11) korrespondierend aufnehmbar sind, ohne dass sich die zwei Bänder (2') gegenseitig räumlich behindern,
so dass im aufgenommenen Zustand des Bandes (2') oder der Bänder (2') eine oder zwei verstellbare Ösen (4) zur Aufnahme von einem oder zwei Fingern vorausgebildet sind, welche durch ein Bewegen des Bandes (2') oder der Bänder (2) durch die Ausnehmung (11) oder die Ausnehmungen (11) in ihrem Durchmesser veränderbar sind.

4. Fingerorthese gemäß Anspruch 1, 2 oder 3 **dadurch gekennzeichnet, dass** die Noppen oder Einkerbungen (21) auf der Außenseite des, die Öse (4) bildenden Bandes (2; 2') angeordnet sind.

5. Fingerorthese gemäß einem oder mehrerer der Ansprüche 1, 2, oder 4, **dadurch gekennzeichnet, dass** der Abstand der Noppen oder Einkerbungen (21) zueinander regelmäßig ist.

6. Fingerorthese gemäß Anspruch 4 und 5, **dadurch gekennzeichnet, dass** der Steg (1'; 1") und das Band (2; 2') anhand patientenspezifischer Computertomographiedaten per 3D-Druck- oder Spritzgussverfahren aus Kunststoffpulver hergestellt sind.

7. Fingerorthese gemäß einem oder mehrerer der Ansprüche 1 bis 6, **dadurch gekennzeichnet, dass** der Kunstsoff mit Wirkstoffen versehen ist, welche aus dem Kunststoff in den Finger diffundieren können.

8. Fingerorthese gemäß einem oder mehrerer der Ansprüche 1 bis 7, **dadurch gekennzeichnet, dass** der Steg (1'; 1") an seinen beiden Enden dichotom in je eine zangenartig-runde Form übergeht, wobei die Ausnehmungen oder Laschen (11) sich gegenüber liegen und mit den Bändern (2 oder 2') die Ösen (4) bilden, so dass ein Relative Motion Splint mit brillenartiger Form ausgebildet ist.

9. Verfahren zur Herstellung einer Fingerorthese gemäß einem oder mehrerer der Ansprüche 1 bis 8 bei dem patientenspezifische Daten eines oder mehrerer Patientenfinger durch ein optisches Scanverfahren ermittelt werden, die ermittelten patientenspezifischen Daten zur Steuerung und Regelung eines 3D-Druck- oder Spritzgussverfahrens mit patientenspezifischen Daten des Stegs (1'; 1") und Daten für die Position der Ausnehmungen oder Laschen (11) am Steg (1'; 1") eingesetzt werden, um den Steg (1'; 1") und das Band (2') aus Kunststoffmaterial zu drucken, oder die Ausnehmungen oder Laschen (11) mit dem Band (2') oder die Bänder (2 oder 2') zu versehen, so dass eine verstellbare Ösen (4) oder zwei verstellbare Ösen (4) zur Aufnahme eines oder zwei Patientenfinger vorausgebildet sind.

10. Verwendung einer Fingerorthese gemäß einem oder mehrerer der Ansprüche 1 bis 8 als Frühmobilisations- Blockungs- oder Entlastungsschienen der Finger eines Patienten,

11. Verwendung einer Fingerorthese gemäß einem oder mehrerer der Ansprüche 1 bis 8 als Relative Motion Splint.
